Europäisches Patentamt

(19) European Patent Office

Office européen des brevets

(11) Numéro de publication : **0 243 278 B1**

(12) **FASCICULE DE BREVET EUROPEEN**

(45) Date de publication du fascicule du brevet :
20.02.91 Bulletin 91/08

(51) Int. Cl.⁵ : **A61K 9/08, A61K 31/475**

(21) Numéro de dépôt : 87400960.8

(22) Date de dépôt : 24.04.87

(54) Solution aqueuse stable de sulfate de vincristine.

(30) Priorité : 25.04.86 FR 8606030

(43) Date de publication de la demande :
28.10.87 Bulletin 87/44

(45) Mention de la délivrance du brevet :
20.02.91 Bulletin 91/08

(84) Etats contractants désignés :
AT BE CH DE ES FR GB GR IT LI LU NL SE

(56) Documents cités :
DE-A- 3 324 964
GB-A- 1 254 057
US-A- 3 749 784
REMINGTON'S PHARMACEUTICAL SCIEN-
CES, 14ième édition, 1970, pages 1523-1525,
Mack Publishing Co., Easton, Pennsylvania,
US

(73) Titulaire : PIERRE FABRE MEDICAMENT
125, Rue de la Faisanderie
F-75116 Paris (FR)

(72) Inventeur : Leverd, Elie
Chemin de Cazers-Bas Lambert
F-81100 Castres (FR)
Inventeur : Bauer, Michel
150 Chemin des Fourches-Hautes
F-81100 Castres (FR)
Inventeur : Basquin, Serge
14, rue Borrel
F-81100 Castres (FR)

(74) Mandataire : Ahner, Francis et al
CABINET REGIMBEAU, 26, avenue Kléber
F-75116 Paris (FR)

## Description

La présente invention concerne une solution aqueuse stable de sulfate de vincristine.

Les alcaloïdes des Vinca sont très utilisés en thérapeutique médicale. Parmi eux, les dimères de structure indolique, en particulier la vincristine, démontrent des propriétés intéressantes dans le domaine de la cancérologie. Plus particulièrement, le sulfate de vincristine, présenté sous forme de préparation injectable, administré par voie intra-veineuse, donne d'excellents résultats dans ce domaine.

Jusqu'à ces dernières années, cette préparation injectable était toujours présentée sous la forme d'un lyophilisat, essentiellement en raison des problèmes liés à l'instabilité de la molécule en solution aqueuse.

Des études plus récentes, par exemple illustrées par GB-A-2 125 292, portent sur des compositions stables aqueuses d'alcaloïdes, comme la vincristine, qui contiennent à titre d'excipients, des polyols, un tampon acétate pour fixer la valeur du pH de la composition dans un domaine particulier, ainsi que des agents anti-microbiens.

Le but de la présente invention a été de mettre au point une nouvelle formulation injectable de sulfate de vincristine en solution aqueuse, dont la stabilité soit particulièrement élevée.

Cette nouvelle formulation, objet de la présente invention, consiste en une solution aqueuse stable de sulfate de vincristine, caractérisée en ce qu'elle comprend ;

a) une quantité thérapeutiquement efficace de sulfate de vincristine ;

b) du glycocolle à raison de 0,1 % à 2,2 % (p/v) ;

c) un tampon phosphate 0,05 M à 0,2 M, à base de phosphate monopotassique, et de l'acide phosphorique, pour fixer le pH de la solution à $4,15 \pm 0,15$ ;

d) un conservateur anti-microbien en quantité efficace et pharmacologiquement compatible ; et

e) de l'eau pour préparation injectable.

Le premier constituant essentiel de la composition aqueuse selon l'invention, est le glycocolle ou glycine. Il s'agit d'un amino-acide, l'acide amino-acétique, qui est destiné à ajuster l'isotonicité de la solution injectable. Le glycocolle intervient dans la composition selon l'invention à raison de 0,1 jusqu'à 2,2 % (p/v), la limite supérieure de 2,2 % poids/volume correspondant à l'obtention d'une solution qui est iso-osmotique avec le sang.

Le second constituant essentiel de la composition selon l'invention est un tampon phosphate, à base de phosphate monopotassique, associé à de l'acide phosphorique. Le tampon phosphate peut présenter une molarité comprise entre 0,05 M et 0,2 M et de préférence une molarité de l'ordre de 0,1 M. Ce couple tampon phosphate, acide phosphorique est destiné à fixer le pH de la solution aqueuse à une valeur de pH = $4,15 \pm 0,15$, ce qui représente le pH de stabilité optimum de la composition à + 4°C. Les résultats analytiques indiqués ci-après démontrent clairement que lorsqu'on s'écarte de cette valeur, la stabilité de la solution décroît très rapidement.

La composition selon l'invention contient en outre des conservateurs anti-microbiens. Ils peuvent être utilisés seuls ou en association entre eux, en quantité efficace et pharmacologiquement compatible. Ces conservateurs anti-microbiens seront par exemple avantageusement choisis parmi la liste figurant ci-après et précisant en outre les teneurs maximales d'utilisation :

| | |
|---|---|
| – parahydroxybenzoate de méthyle | 1,5 °/₀₀ (p/v) |
| – parahydroxybenzoate de propyle | 1,5 °/₀₀ (p/v) |
| – phénol | 5 °/₀₀ (p/v) |
| – crésols | 3 °/₀₀ (p/v) |
| – dérivés mercuriels | 0,1 °/₀₀ (p/v) |
| – alcool benzylique | 10 °/₀₀ (p/v). |

A titre d'illustration de l'objet de la présente invention, on indiquera ci-après un exemple particulier de solution aqueuse stable de sulfate de vincristine qui répond à la formule :

| | | |
|---|---|---|
| – sulfate de vincristine | 1 | mg |
| – parahydroxybenzoate de méthyle | 1,275 | mg |
| – parahydroxybenzoate de propyle | 0,225 | mg |
| – glycocolle | 18,900 | mg |
| – tampon phosphate 0,1 M pH = 4,5 | 0,200 | ml |
| – acide phosphorique | q.s.  pH = 4,15 | |
| – eau pour préparations injectables | q.s.p.  1 | ml. |

La préparation d'une telle solution n'appelle aucun commentaire particulier étant données les caractéristiques de solubilité dans l'eau de chacun de ces constituants. Cependant, à cause de la thermosensibilité du sulfate de vincristine, la stérilisation de la solution est assurée par filtration sur membrane de porosité 0,2 µm.

Le récipient de conditionnement peut être une ampoule de verre blanc ou brun, un flacon également en verre et muni d'un bouchon en élastomère judicieusement choisi.

Etude analytique en fonction de la température et du pH-Optimisation dans le cadre du modèle arrhénien.

L'étude de la dégradation forcée du sulfate de vincristine en solution a été suivie par chromatographie liquide haute performance.

Les conditions ont été les suivantes :
– Colonne : microbondapak[(R)] (WATERS) L = 30 cm di = 4,2 mm remplie par de l'octadécylsilane (C 18 – 10 µm).
– Eluant :

| | |
|---|---|
| eau | 650 ml |
| acétonitrile | 350 ml |
| chlorure de potassium | 7.45 g |
| acide chlorhydrique N | 15 ml. |

– Débit : 1 ml/min
– Longueur d'onde de détection : 276 nm.

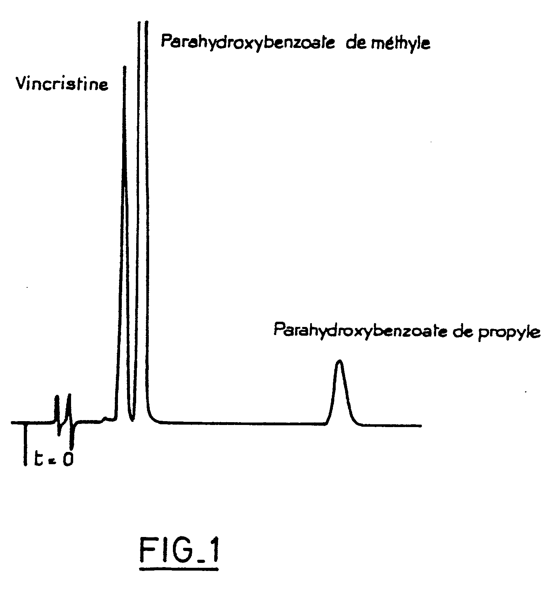

La figure 1 représente le chromatogramme type de la solution injectable préparée suivant la formule citée ci-dessus.

L'analyse des points expérimentaux montre que la dégradation du sulfate de vincristine en solution suit une loi cinétique du premier ordre.

$$\text{Log} \frac{C}{C_0} = -kt \quad (1)$$

avec C = concentration en mole/l de sulfate de vincristine intact à l'instant (t)
$C_0$ = concentration initiale en mole/l de sulfate de vincristine
t = le temps en secondes
k = constante cinétique de dégradation en $s^{-1}$ du sulfate de vincristine. Cette constante k est fonction de la température et du pH de la solution considérée. Elle obéit à la loi d'ARRHENIUS classique :

$$\text{Log } k (T,pH) = \text{Log } k_0 (T,pH) - \frac{E_A (pH)}{RT} \quad (2)$$

où : $E_A$ est l'énergie d'activation du processus de dégradation en kcal.mole$^{-1}$ qui est fonction du pH de la solution considérée. $k_0$ est le facteur de fréquence en $s^{-1}$ relié à l'entropie d'activation de l'état de transition du processus dégradatif qui est également fonction du pH de la solution considérée.
T = la température absolue en degré KELVIN (°K)
R = la constante des gaz parfaits égale à 0,00198 en Kcal.mole$^{-1}$ . °K$^{-1}$.

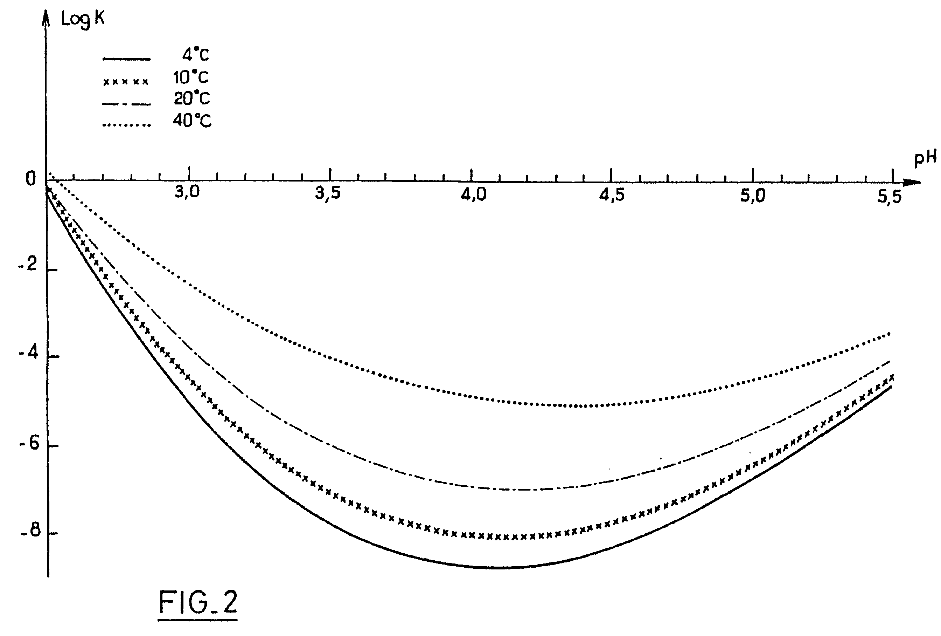

La figure 2 représente la variation (*) de K en fonction du pH pour diverses températures (4°C, 10°C, 20°C, 40°C).

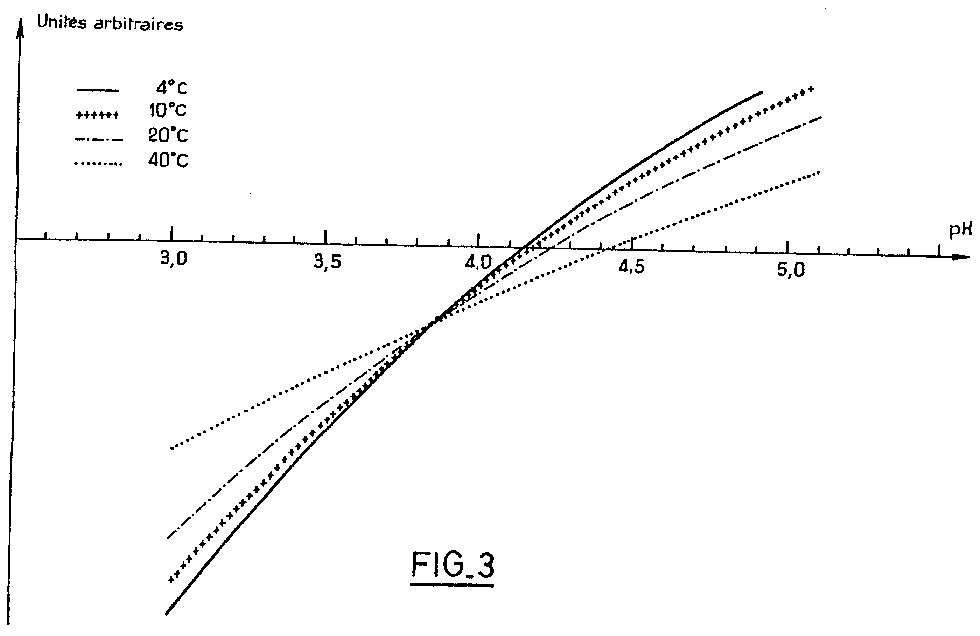

La figure 3 représente la courbe dérivée :

$$\frac{\delta \, \text{Log K (T, pH)}}{\delta \, \text{pH}}$$

en fonction du pH et pour la même série de température.

On voit que pour une conservation à 4°C, l'optimum ($\frac{\delta \, \text{Log K}}{\delta \, \text{pH}} = 0$) est atteint pour une valeur de pH égale à 4,15 ± 0,15.

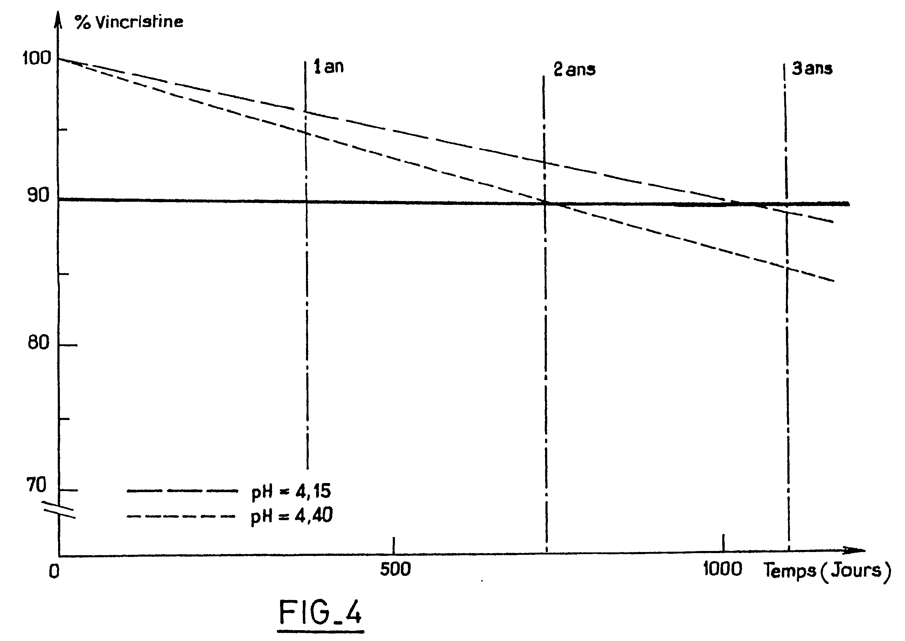

A partir de ces conditions, on peut prévoir un profil de décroissance de la teneur en sulfate de vincristine dans la solution injectable, conservée à 4°C, ayant l'allure représentée sur la figure 4 (environ 7,5 % de dégradation sur 2 ans).

A titre de comparaison, on donne sur le même graphique l'évolution obtenue pour une solution injectable tamponnée à 4,4 et maintenue à 4°C.

(*) N.B. : Les courbes présentées résultent de l'utilisation d'une formule empirique polynomiale du type :

$$y = a_3 x^3 + a_2 x^2 + a_1 x + a_0,$$

les paramètres $a_0$ $a_1$ $a_2$, $a_3$ étant ajustés par la méthode des moindres carrés à partir des valeurs expérimentales.

**Revendications**

1. Solution aqueuse stable de sulfate de vincristine, destinée à être administrée par voie parentérale, caractérisée en ce qu'elle comprend :

a) une quantité thérapeutiquement efficace de sulfate de vincristine ;

b) du glycocolle à raison de 0,1 % à 2,2 % (p/v) ;

c) un tampon phosphate 0,05 M à 0,2 M, à base de phosphate monopotassique, et de l'acide phosphorique, pour fixer le pH de la solution à 4,15 ± 0,15 ;

d) un conservateur anti-microbien en quantité efficace et pharmacologiquement acceptable ; et

e) de l'eau pour préparation injectable.

2. Solution selon la revendication 1, caractérisée en ce que le conservateur est choisi parmi la liste des produits suivants, précisant les teneurs maximales d'utilisation :

| | | |
|---|---|---|
| - parahydroxybenzoate de méthyle | 1,5 | °/₀₀ (p/v) |
| - parahydroxybenzoate de propyle | 1,5 | °/₀₀ (p/v) |
| - phénol | 5 | °/₀₀ (p/v) |
| - crésols | 3 | °/₀₀ (p/v) |
| - dérivés mercuriels | 0,1 | °/₀₀ (p/v) |
| - alcool benzylique | 10 | °/₀₀ (p/v). |

3. Solution selon l'une des revendications 1 et 2, caractérisée en ce qu'elle répond à la composition suivante :

| | | |
|---|---|---|
| - sulfate de vincristine | 1 | mg |
| - parahydroxybenzoate de méthyle | 1,275 | mg |
| - parahydroxybenzoate de propyle | 0,225 | mg |
| - glycocolle | 18,900 | mg |
| - tampon phosphate 0,1 M pH = 4,5 | 0,200 | ml |
| - acide phosphorique q.s. | pH = 4,15 | |
| - eau pour préparations injectables q.s.p. | 1 | ml. |

## Claims

1. A stable aqueous solution of vincristine sulphate for parenteral administration, characterised in that it comprises :

a) a therapeutically effective quantity of vincristine sulphate ;

b) 0.1 to 2.2% (w/v) of glycocol ;

c) a 0.05 M to 0.2 M phosphate buffer based on monopotassium phosphate and phosphoric acid, to keep the pH of the solution at 4.15 ± 0.15 ;

d) an efficient quantity of a pharmacologically acceptable antimicrobial preservative, and

e) water for an injectable preparation.

2. A solution according to claim 1, characterised in that the preservative is chosen from among the following list of products, specifying the maximum contents to be used :

|  | parts per 1000 |
| --- | --- |
| Methyl parahydroxybenzoate | 1.5 (w/v) |
| Propyl parahydroxybenzoate | 1.5 (w/v) |
| Phenol | 5 (w/v) |
| Cresols | 3 (w/v) |
| Mercury derivatives | 0.2 (w/v) |
| Benzyl alcohol | 10 (w/v). |

3. A solution according to claim 1 or 2, characteriesd in that it has the following composition :

| Vincristine sulphate | 1 | mg |
| --- | --- | --- |
| Methyl parahydroxybenzoate | 1.275 | mg |
| Propyl parahydroxybenzoate | 0.225 | mg |
| Glycocol | 18.900 | mg |
| Phosphate buffer, 0.1 M, pH = 4.5 | 0.200 | ml |
| Phosphoric acid | q.s. pH = 4.15 | |
| Water for injectable preparations | q.s.p. 1 | ml |

## Ansprüche

1. Wäßrige stabile Lösung von Vincristinsulfat, bestimmt zur Verabreichung auf parenteralem Wege, dadurch gekennzeichnet, daß sie umfaßt :

a) eine therapeutisch wirksame Menge von Vincristinsulfat ;

b) Glykokoll in einer Menge von 0,1 % bis 2,2 % (Gew/Vol) ;

c) einen 0,05 M bis 0,2 M Phosphatpuffer auf der Basis von Monokaliumphosphat und Phosphorsäure, um den pH-Wert der Lösung auf 4,15 ± 0,15 zu fixieren ;

d) ein antimikrobielles Konservierungsmittel in wirksamer und pharmakologisch brauchbarer Menge ; und

e) Wasser für ein injizierbares Präparat.

2. Lösung nach Anspruch 1, dadurch gekennzeichnet, daß das Konservierungsmittel ausgewählt ist aus der Liste folgender Produkte, in der der maximal zu verwendende Gehalt angegeben ist :

EP 0 243 278 B1

| | | |
|---|---|---|
| - para-Hydroxybenzoesäure-methylester | 1,5 | $^o$/oo (Gew/Vol) |
| - para-Hydroxybenzoesäure-propylester | 1,5 | $^o$/oo (Gew/Vol) |
| - Phenol | 5 | $^o$/oo (Gew/Vol) |
| - Kresole | 3 | $^o$/oo (Gew/Vol) |
| - quecksilberhaltige Derivate | 0,1 | $^o$/oo (Gew/Vol) |
| - Benzylalkohol | 10 | $^o$/oo (Gew/Vol) . |

3. Lösung nach einem der Ansprüche 1 und 2, dadurch gekennzeichnet, daß sie folgender Zusammensetzung entspricht :

| | | |
|---|---|---|
| - Vincristinsulfat | 1 | mg |
| - para-Hydroxybenzoesäure-methylester | 1,275 | mg |
| - para-Hydroxybenzoesäure-propylester | 0,225 | mg |
| - Glykokoll | 18,900 | mg |
| - Phosphatpuffer 0,1 M pH = 4,5 | 0,200 | ml |
| - Phosphorsäure | q.s. | pH = 4,15 |
| - Wasser zur Herstellung injizierbarer Präparate | q.s.p 1 | ml . |

6

Vincristine

Parahydroxybenzoate de méthyle

Parahydroxybenzoate de propyle

t = 0

## FIG.1

FIG.2

EP 0 243 278 B1

FIG_3

EP 0 243 278 B1

FIG_4

EP 0 243 278 B1